# EUROPEAN PATENT SPECIFICATION

(11) **EP 1 916 310 B1**
(45) Date of publication and mention of the grant of the patent: **26.09.2018**
(21) Application number: 06747306.6
(22) Date of filing: 09.06.2006
(51) Int. Cl.: G01N 33/50, G01N 33/569, C12Q 1/02, C12N 1/00

(54) **METHOD FOR ANALYSIS OF NKT CELL FUNCTION**
VERFAHREN ZUR ANALYSE DER NKT-ZELLFUNKTION
PROCEDE D'ANALYSE DE LA FONCTION D'UNE CELLULE NKT

(30) Priority: 17.06.2005 JP 2005178284
(43) Date of publication of application: 30.04.2008
(73) Proprietor: RIKEN, Wako-shi, Saitama 351-0198 (JP)
(72) Inventor: FUJII, Shin-ichiro, c/o RIKEN Yokohama Institute, Yokohama-shi, Kanagawa 230-0045 (JP); SHIMIZU, Kanako, c/o RIKEN Yokohama Institute, Yokohama-shi, Kanagawa 230-0045 (JP)
(74) Representative: J A Kemp
(86) International application number: PCT/JP2006/312079
(87) International publication number: WO 2006/135032

(56) References cited:
- US-A1- 2003 153 073
- BENLAGHA KAMEL ET AL: "In vivo identification of glycolipid antigen-specific T cells using fluorescent CD1d tetramers" JOURNAL OF EXPERIMENTAL MEDICINE, vol. 191, no. 11, 5 June 2000 (2000-06-05), pages 1895-1903, XP002491440 ISSN: 0022-1007
- GODFREY DALE I ET AL: "Going both ways: immune regulation via CD1d-dependent NKT cells" JOURNAL OF CLINICAL INVESTIGATION, vol. 114, no. 10, November 2004 (2004-11), pages 1379-1388, XP002491441 ISSN: 0021-9738
- SHIMIZU KANAKO ET AL: "Evaluation of the function of human invariant NKT cells from cancer patients using alpha-galactosylceramide-loaded murine dendritic cells" JOURNAL OF IMMUNOLOGY, vol. 177, no. 5, September 2006 (2006-09), pages 3484-3492, XP002491442 ISSN: 0022-1767
- BROSSAY L. ET AL.: 'CD1d-mediated recognition of an alpha-Galactosylceramide by natural killer T cells is highly conserved through mammalian evolution' J. EXP. MED. vol. 188, 1998, pages 1521 - 1528, XP002996702
- FUJII S.: 'NKT Saibo to Jujo Saibokan ni okeru Kasseika to Seigyo Kiko' EXPERIMENTAL MEDICINE vol. 22, no. 5, 25 March 2004, pages 56 - 63, XP003007245
- FUJII S. ET AL.: 'Prolonged IFN-gamma-producing NKT response induced with alpha-galactosylceramide-loaded DCs' NATURE IMMUNOLOGY vol. 3, no. 9, 2002, pages 867 - 874, XP003007246
- K Yamada ET AL: "Human anti-porcine xenogeneic T cell response. Evidence for allelic specificity of mixed leukocyte reaction and for both direct and indirect pathways of recognition", The Journal of Immunology, 1 December 1995 (1995-12-01), page 5249, XP055169160, UNITED STATES Retrieved from the Internet: URL:http://www.jimmunol.org/cgi/content/ab stract/155/11/5249
- VAN DER VLIET H J J ET AL: "Potent expansion of human natural killer T cells using alpha-galactosylceramide (KRN7000)-loaded monocyte-derived dendritic cells, cultured in the presence of IL-7 and IL-15", JOURNAL OF IMMUNOLOGICAL METHODS, ELSEVIER SCIENCE PUBLISHERS B.V.,AMSTERDAM, NL, vol. 247, no. 1-2, 1 January 2001 (2001-01-01), pages 61-72, XP004228399, ISSN: 0022-1759, DOI: 10.1016/S0022-1759(00)00272-6

## Description

### Technical Field

The present invention relates to a method of functional analysis of NKT cell, particularly, a method of functional analysis of human NKT cell, which utilizes CDld-expressing antigen presenting cells derived from a heterologous animal.

### Background Art

NKT cell constitutes a lymphocyte linage having both characteristics of T cell and NK cell, and is activated by an antigen presented on CD1d, a MHC class I-like molecule, to exhibit actions such as antitumor action. The antigen presented on CDld and which strongly activates a NKT cell, includes, for example, glycolipid α-galactosylceramide (α-GalCer). The primary NKT cell can be stimulated by an antigen presenting cell (APC) loaded with an antigen such as α-GalCer to differentiate and grow. As such APCs, for example, macrophage, immature or mature dendritic cell (DC) and the like are known. The present inventors previously reported that an α-GalCer-pulsed mature DC, above all, strongly activated a primary NKT cell in the peripheral blood (Fujii et al., Journal of Immunological Methods 2003, 272: 147-59).

It is known that, in patients with an immune-associated disease such as cancer, virus infection, autoimmune disease and the like, a decrease in the number or function of NKT cells is associated with the pathology (e.g. progression of disorder). Accordingly, the development of a method of standardizing the procedure is desired, which renders NKT cell analysis an index of progression of such diseases. Also in the NKT cell therapy being currently performed, the development of a low frequency NKT cell analysis method is desired.

Examples of the functional analysis methods of NKT cell till date include the following reports.
1) ELISPOT assay where the function of NKT cell is monitored using α-GalCer-pulsed APCs (Fujii et al., Journal of Immunological Methods 2003, 272: 147-59; Fujii et al., British Journal of Haematology 2003, 122: 617-22; Moiling et al., International Journal of Cancer 2005, 116: 87-93).
2) Tetramer assay where the functional analysis of NKT cell is performed by intracellular cytokine staining using CDld tetramers (Gumperz et al., Journal of Experimental Medicine 2002, 195: 625-636).
3) A method comprising collecting NKT cells from tested samples, and constructing a NKT cell line, after which the functional analysis thereof is performed (Tahir et al., Journal of Immunology 2001, 167: 4046-50; Yanagisawa et al., Journal of Immunology 2002, 168: 6494-9).

However, the above-mentioned 1) - 3) respectively have the following problems.

As to the method 1), since the detection of peripheral blood NKT cells is difficult when the cell is of low frequency (< 0.05%), the functional analysis of NKT cell in a tested sample with low frequency of peripheral blood NKT cell (e.g., cancer patient) is problematically difficult. Furthermore, since the results of functional analysis of NKT cell by ELISPOT assay vary depending on autologous APCs, there is a problem in that a genuine functional analysis of NKT cell per se, which is independent of autologous APCs, is unattainable.

As to the method 2), since the detection sensitivity of the assay vary depending on the detection sensitivity of flow cytometry, a problem occurs in that the functional analysis of peripheral blood NKT cell is difficult when the cell is of low frequency (< 0.05%). Furthermore, since the results of functional analysis of NKT cell by Tetramer assay vary depending on autologous APCs, there is a problem in that a genuine functional analysis of NKT cell per se, which is independent of autologous APCs, is unattainable.

As to the method 3), since it requires a long-term cultivation, an influence of secondary factors such as IL-12 etc. is unavoidable, which problematically affords only the analysis of a particular NKT cell line that has survived the long-term cultivation.

Brossay et al, J. Exp. Med. 188(8), 1521-1528, 1998 reports that mouse NK T cells can recognize human CDld as well as mouse CD1, and that human NK T cells also recognize both CD1 homologues. Further, Brossay *et al* report that mouse or human CD1 molecules can present the glycolipid α-galactosylceramide (α-GalCer) to NK T cells from either species. Human T cells, preselected for invariant Vα24 TCR expression, uniformly recognized α-GalCerpresented by either human CDld or mouse CD1. In addition, culture of human peripheral blood cells with α-GalCerled to the expansion of NK T cells with an invariant (Vα24+) TCR and the release of large amounts of cytokines.

US 2003/0153073 A1 discloses methods for stimulating the proliferation of human T cells *in vitro* comprising repeatedly culturing donor T cells in the presence of antigen presenting cells, an antigen, a cell survival factor and serum for at least 7 days under conditions stimulating proliferation of the T cells.

Benlagha et al, J. Exp. Med. 191, 11, 1895-1903, 2000 reports the generation and use of mouse CD1d1-glycolipid tetramers to visualize CD1d-restricted T cells. In contrast with previous BIAcore-based estimates of very short half-lives for CD1d-glycolipid complexes, it was found that the dissociation rate of several different CD1d-glycolipid complexes was very slow. Fluorescent tetramers of mouse CD1d1 complexed with α-galactosylceramide (α-GalCer), the antigen recognized by mouse Vα14-Jα281/Vβ8 and human Vα24-JαQ/Vβ11 natural killer T (NKT) cell T cell receptors (TCRs). This allowed the accurate description, based on TCR specificity, of the entire population of NKT cells *in vivo* and identification of previously unrecognised population of NK1.1-negative "NKT" cells which expressed a different pattern of integrins.

Hans J. J. van der Vliet et al, J. Immun. Methods 247, 61-72, 2001 reports a 7-day co-culture of peripheral blood mononuclear cells and α-GalCer KRN7000-loaded mature monocyte derived dendritric cells of human origin in the presence of interleukin-7 and interleukin-15. They observed up to 76-fold expansion of Vα24⁺ Vβ11⁺-T cells. The expanded Vα24⁺ Vβ11⁺-T cells expressed the cytotoxic molecule granzyme B, showed negible expression of Fas ligand and could be induced to express high levels of interferon-γ, while retaining the capacity to produce IL-4.

### Disclosure of the Invention

An object of the present invention is to provide a method of functional analysis of NKT cell, which is more superior to the conventional methods, which enables functional analysis of low frequency NKT cell, does not depend on the function of autologous APCs, and can avoid an influence of secondary factors, and the like.

As a result of the diligent examination, the present inventors have found that CDld expressing-APCs derived from a heterologous animal only needs to be used for functional analysis of human NKT cell, which is independent of the function of autologous APCs, that the above-mentioned analysis system enables an assay superior in the accuracy and reproducibility since it can avoid an influence of secondary factors such as cytokines etc., that since NKT cells can be grown by long-term cultivation while maintaining the proportional relationship between the number of NKT cells before and after the cultivation, the long-term cultivation improves the detection sensitivity of the assay, thus enabling a functional analysis of low frequency NKT cell, and the like. Based on these findings and the like, the present inventors have succeeded in the construction of a more superior functional analysis system of NKT cell and resolved the above-mentioned problems.

Accordingly, the present invention provides the following:
[1] a method of functional analysis of NKT cell in human, which comprises:
   (a) cocultivating a mononuclear cell population containing a human NKT cell derived from human peripheral blood with a mature dendritic cell derived from a rodent, wherein the mature dendritic cell is a cell pulsed with a CDld ligand, and
   (b) evaluating the functionality of NKT cell based on the number of NKT cells and/or a protein specific to functional NKT cells or a transcription product encoding same as an index;
[2] the method of [1] above, wherein the CDld ligand is α-GalCer;
[3] the method of [1] above, wherein the mononuclear cell population is a macrophage-depleted mononuclear cell population;
[4] the method of [1] above, wherein the substance specific to functional NKT cells is IFN-γ;
[5] the method of [1] above, wherein the evaluation of the functionality of NKT cell based on the number of NKT cells and/or a protein specific to functional NKT cells or a transcription product encoding same as an index is based on a measurement of frequency of cell that expresses the protein specific to functional NKT cells or a transcription product encoding same;
[6] the method of [1] above, wherein the rodent is a mouse;
[7] the method of [1] above, which is an analysis method of human that shows NKT cell frequency of about 0.05% - about 0.001% in peripheral blood;
[8] the method of [1] above, which is a method for monitoring an immune therapy, or determination of the pathology or prognosis in a patient with a disease selected from the group consisting of cancer, autoimmune disease, allergic disease and virus infection.

The present invention is useful because it can afford a method of functional analysis of NKT cell, which is more superior to the conventional methods, which enables functional analysis of low frequency NKT cell, does not depend on the function of autologous APCs, and can avoid an influence of secondary factors and the like.

### Brief Description of the Drawings

Fig. 1 shows an expression profile of human/mouse dendritic cell. DC: dendritic cell
Fig. 2 shows a comparison of the activation rates obtained using dendritic cells in a high frequency (%NKT > 0.05%) NKT cell-containing group. For each system, the experiment was performed using PBMCs diluted 100-, 30- or 10-fold. DC: dendritic cell G: α-GalCer
Fig. 3 shows a comparison of the activation rates obtained using dendritic cells in a middle frequency (0.01% < %NKT < 0.05%) NKT cell-containing group. For each system, the experiment was performed using PBMCs diluted 100-, 30- or 10-fold. DC: dendritic cell G: α-GalCer
Fig. 4 shows a comparison of the activation rates obtained using dendritic cells in a low frequency (%NKT < 0.01%) NKT cell-containing group. For each system, the experiment was performed using PBMCs diluted by 100-, 30- or 10-fold. DC: dendritic cell G: α-GalCer
Fig. 5 shows the results of mixed lymphocyte reaction (MLR) with autologous, allogeneic or heterologous dendritic cells.
Fig. 6 shows maturation of dendritic cell phenotype by autologous NKT cells. Isotype shows the results when a mouse anti-human IgG1-PE antibody was used.
Fig. 7 shows alteration in the cytokine production ability of dendritic cell by autologous NKT cells.
Fig. 8 shows the mode of interaction between an autologous or heterologous dendritic cell and an NKT cell.
Fig. 9 shows antigen-specific activation of human NKT cell by heterologous dendritic cells. hDC: human dendritic cell mDC: mouse dendritic cell G: α-GalCer hNKT: human NKT cell CK: cytokine cocktail
Fig. 10 shows antigen-specific direct proportional growth of human NKT cell by heterologous dendritic cells.
Fig. 11 demonstrates antigen-specific growth and IFN-γ production of peripheral blood NKT cell from a healthy subject by heterologous dendritic cells. DC: dendritic cell G: α-GalCer
Fig. 12 demonstrates antigen-specific growth and IFN-γ production of peripheral blood NKT cell from plural healthy subjects (including low frequency NKT cell-containing group) by heterologous dendritic cells.
Fig. 13 shows the analysis results of a representative case of chronic myeloid leukemia (CML) patient, for whom growth and IFN-γ producibility of NKT cell by heterologous dendritic cell were demonstrated. DC: dendritic cell G: α-GalCer
Fig. 14 shows the analysis results of 10 cases of CML patients, for whom growth and IFN-γ producibility of NKT cell by heterologous dendritic cells were demonstrated. As chronic myeloid leukemia patients, patients receiving treatment with imatinib were adopted as the subjects. PR: a patient group that showed partial remission due to the treatment with imatinib CR: a patient group that showed complete remission due to the treatment with imatinib

### Best Mode for Embodying the Invention

The present invention provides a method of functional analysis of human NKT cell. The method of the present invention can comprise, for example, the following (a) and (b) :
(a) cocultivating a mononuclear cell population containing a human NKT cell derived from human peripheral blood with a mature dendritic cell derived from a rodent, wherein the mature dendritic cell is a cell pulsed with a CDld ligand, and
(b) evaluating the functionality of NKT cell based on the number of NKT cells and/or a protein specific to functional NKT cells or a transcription product encoding same as an index.

While the cocultivation of the above-mentioned (a) can be performed in any manner permitting activation, by the mature dendritic cell, of the NKT cell contained in the mononuclear cells derived from the human peripheral blood, it is performed using a mature dendritic cell pulsed by a CDld ligand. This leads to the presentation of the CDld ligand on the mature dendritic cell, and then to an efficient activation, by the cell presenting the CDld ligand, of the NKT cell contained in mononuclear cells derived from the human peripheral blood, which ultimately improves the sensitivity of the method of the present invention.

When used in the present specification, the "heterologous animal" means a nonhuman animal. In the method of the present invention, the mature dendritic cell derived from a rodent needs to be able to activate a human NKT cell. As such heterologous animals, for example, birds such as chicken, or mammals such as rodents (e.g., mouse, rat, rabbit, guinea pig, hamster) or nonrodents (bovine, horse, goat, sheep, swine, dog, cat, monkey) can be mentioned. Also, in the method of the present invention, to keep the background noise low to realize a functional analysis of NKT cell superior in the accuracy and reproducibility, it is important that no immune response that may reduce accuracy and reproducibility of the method of the present invention (e.g., production of a Th1-type cytokine such as IFN-γ by T cells) occurs between a human immunocompetent cell (e.g., T cell) contained in human peripheral blood and a mature dendritic cell derived from a heterologous animal. For this end, the heterologous animal is a rodent from among those mentioned above, and more preferably mouse.

When used in the present specification, a "CD1d-expressing antigen presenting cell (CD1d-expressing APC)" means a cell that bears CDld on its cell surface and can activate a NKT cell. An example of a CDld-expressing APC is a mature dendritic cell. In addition, as the mature dendritic cell, for example, a cell differentiation-induced from a precursor cell thereof (e.g., bone marrow cell), or a differentiated cell found in peripheral blood can be used. A differentiation-induced cell is preferable, since its function can be maintained even after freeze preservation, and the stability of the cell can be maintained even after cultivation. Induction and maturation of the dendritic cell can be performed by a method known per se, for example, after removal of unnecessary cells from bone marrow cells (e.g., macrophage, B cell, T cells, NK cell), adding GM-CSF and then adding LPS for 16 hr at the 7th day (see, e.g., Fujii et al., Nat. Immunol. 3: 867-74 (2002)).

A dendritic cell that is differentiation-induced from a bone marrow cell and matured is more preferable since it is superior in the detection sensitivity of functional NKT cells and versatility, it can maintain its function even on freeze preservation, it can be easily obtained and can be acquired in abundance, and the like.

While the induced dendritic cell can be obtained by cultivation of a bone marrow cell in the presence of a given differentiation-inducing factor such as GM-CSF (e.g., GM-CSF from a rodent such as mouse), or by isolation of a dendritic cell from spleen, and the like, the one obtained by cultivation of a bone marrow cell in the presence of GM-CSF can be preferably used, since it is superior in the stability of a surviving cell.

Maturation of a dendritic cell can be performed by cultivation of an immature dendritic cell in the presence of, for example, a given maturation factor. As such maturation factor, for example, LPS, CD1d ligand (mentioned below), anti-CD40 antibody, TNF-α, CpG-DNA can be mentioned, and LPS is preferable since it is superior in the selectivity for a dendritic cell, economical, superior in the stability, and so on.

When used in the present specification, the "CD1d ligand" means a substance that can be presented on a CD1d-expressing APC to activate a human NKT cell. Examples of the "CD1d ligand" include, but are not particularly limited to, α-GalCer (α-galactosylceramide), α-C-GalCer (α-C-galactosylceramide), iGB3 (isoglobotrihexosylceramide), GD3 (ganglioside 3), GSL-1 (α-linked glucuronic acid), GSL-1'SA (galacturonic acid). α-GalCer and α-C-GalCer are preferable, and α-GalCer is more preferable, since they strongly activate a human NKT cell to further improve detection sensitivity of the method of the present invention. While the concentration of the CDld ligand used for the pulse of the mature dendritic cell is not particularly limited as long as it enables detection of a functional NKT cell, the concentration may be, for example, about 10 - 200 ng/ml, preferably about 100 ng/ml. In the method of the present invention, a mature dendritic cell pulsed by a CDld ligand is used. Accordingly, the method of the present invention can include pulse treatment of the CDld-expressing APC by the CDld ligand simultaneously with or after induction and maturation of the dendritic cell.

While the concentration of the mature dendritic cell used in the cocultivation is not particularly limited as long as it enables activation of the NKT cell to evaluate its functionality, it may be, for example, about 0.1×10⁵ - 1×10⁵ cells/ml, preferably about 0.5×10⁵ cells/ml.

The mononuclear cell derived from human peripheral blood is not particularly limited as long as it is a cell population containing a human NKT cell, which is the analysis target. The human NKT cell may be Vα24- and/or Vβ11-positive cell. In the method of the present invention, while the both of human peripheral blood subjected or not subjected to a given treatment (e.g., cell fractionation treatment) can also be used as long as it contains a human NKT cell, a cell population containing a human NKT cell, from which cell population human CDld-expressing APCs contained in human peripheral blood (e.g., dendritic cell, macrophage, B cell) have been removed (or depleted), is preferably used since a genuine functional analysis of human NKT cell per se is performed while excluding influences from autologous CDld-expressing APCs. Removal (or depletion) of human CDld-expressing APCs from human peripheral blood can be performed by utilizing a marker molecule of the APC. For example, removal of APC can be performed using an antibody against a marker molecule of human dendritic cell (e.g., CD11c, CD83, CD80, CD86, DR) or a marker molecule of human macrophage (e.g., CD14), or using anti-PE antibody-bonded magnetic beads or anti-FITC antibody-bonded magnetic beads. Preferably, the mononuclear cell derived from human peripheral blood may be a mononuclear cell derived from peripheral blood from which macrophages have been removed (Mφ-depletion) using anti-CD14 antibody, or anti-CD14 antibody and anti-CD11c antibody.

The mononuclear cell derived from human peripheral blood to be used in the cocultivation can be prepared from an analysis target human by a method known per se. The number of the mononuclear cells used for the cultivation is not particularly limited as long as it permits functional analysis of the human NKT cell contained therein. There is an individual difference in the proportion of the NKT cells contained in the mononuclear cells, due to the factors of health condition, disease and the like. For example, it has been reported that the number of NKT cells can be remarkably decreased, in cancer patients. In the method of the prevent invention, however, since NKT cells can be grown while preserving the proportional relationship in the number of NKT cells between before and after the cocultivation, the method is advantageous in that the functionality of NKT cells can be analyzed while maintaining the accuracy even when the number of the NKT cells contained in the mononuclear cells is small. From such standpoint, the concentration of mononuclear cells to be used in the cocultivation is, for example, about 1x10⁴ - 10⁶ cells/ml, and preferably about 5x10⁵ cells/ml.

From the below-mentioned Examples, it has also been clarified that NKT cells can be appropriately analyzed by the method of the present invention when the frequency of NKT cells in mononuclear cells is not less than 0.001%. Accordingly, when an analysis is performed using a 96-well plate, for example, for a patient showing an NKT cell frequency of 0.001%, the peripheral blood amount of 10 - 20 ml is considered to be sufficient. It is also one of the advantages of the present invention that a superior functional analysis of NKT cells can be performed accurately with such a small amount of peripheral blood. The method of the present invention can further comprise subjecting the above-mentioned volume of peripheral blood to the above-mentioned (a) with or without a cell fractionation treatment (e.g., removal treatment of CDld-expressing APCs such as macrophage and the like).

The cultivation period is not particularly limited as long as it enables evaluation of the functionality of NKT cell. It has been revealed that NKT cells can be grown by the methodology of above-mentioned (a) while preserving the proportional relationship of the number of NKT cells between before and after the cocultivation. Accordingly, the method of the present invention is advantageous in that, by appropriately setting the cultivation period, the detection sensitivity can be improved while keeping the accuracy, an analysis with a smaller amount of peripheral blood is made possible and the like. When the cultivation is performed with the expectation of such advantages, a preferable cultivation period is not particularly limited as long as it permits growth of NKT cells while preserving the proportional relationship of the number of NKT cells. For example, cultivation of not less than about 7 days, preferably about 7 - 14 days, more preferably about 9 - 12 days, and yet more preferably about 9 - 10 days, can be mentioned.

The medium to be used for the cocultivation can be prepared using the medium used for cultivation of an animal cell as a basal medium. As the basal media, for example, Eagle's MEM medium, αMEM medium, DMEM medium, Ham's medium, RPMI 1640 medium, Fischer's medium, and a mixed medium thereof can be mentioned. The medium can contain, for example, sera (e.g., FCS), serum alternatives (e.g., knockout Serum Replacement (KSR), Chemically-defined Lipid concentrated (manufactured by Gibco), Glutamax (manufactured by Gibco)), fatty acids or lipids, amino acids, vitamins, growth factors, cytokines, antioxidants, 2-mercaptoethanol, pyruvic acid, buffering agents, inorganic salts and the like.

While the incubator to be used for the cocultivation is not particularly limited as long as it permits suspension cultivation of cells, for example, multiwell plates such as a 96-well round bottom plate can be mentioned.

Other culture conditions such as cultivation temperature, CO₂ concentration and the like of the cocultivation can be appropriately set. While the cultivation temperature is not particularly limited, it is, for example, about 30 - 40°C, and preferably about 37°C. The CO₂ concentration is, for example, about 1 - 10%, and preferably about 5%.

IL-2 can be used for the cocultivation. This is because IL-2 is useful in the survival and conservation of NKT cells. While the concentration of IL-2 to be used for the cocultivation is not particularly limited as long as it is preferable for the survival and conservation of NKT cells, it can be, for example, about 5 - 1000 U/ml, preferably about 10 - 100 U/ml, and more preferably about 100 U/ml.

In the above-mentioned (b), the functionality of NKT cell can be evaluated using, as an index, the number of NKT cells and/or a substance specific to functional NKT cells.

The "protein specific to functional NKT cells or a transcription product encoding same" means a protein or transcription product encoding same that can be an index of the functionality of activated NKT cells. The protein specific to functional NKT cells or transcription product encoding same can be a protein such as an intracellular factor or a secretion factor, or a transcription product encoding same. The protein specific to functional NKT cells or transcription product encoding same is, for example, a protein such as interferon-γ (IFN-γ), perforin, granzyme B, or a transcription product encoding same, and interferon-γ (IFN-γ) is preferable since it is convenient and high-sensitive. For evaluation of the functionality of NKT cells, one or more kinds of proteins specific to functional NKT cells or transcription products encoding same can be utilized.

The evaluation of the functionality of NKT cells, for which the number of NKT cells is an index, is performed from the viewpoint of whether or not the number of NKT cells increases by the cocultivation (in other words, whether or not the NKT cells have growth ability). The measurement of the number of NKT cells can be performed using, for example, Vα24 antibody, Vβ11 antibody or anti-invariant NKT antibody. The present evaluation method enables analysis of NKT cells capable of maintaining growth ability.

The evaluation of the functionality of NKT cells, for which a protein or transcription product encoding same specific to functional NKT cells is an index, is performed based on the measurement of the frequency of cells expressing the protein, or measurement of the amount of the protein or transcription product in the culture supernatant. The measurement of the frequency of cells expressing the protein can be performed by, for example, intracellular staining using an antibody against the protein, or a method using a nucleic acid probe capable of detecting a transcription product encoding the protein, or plural amplifiable primers (e.g., primer set) and the like (e.g., RT-PCR such as real-time RT-PCR). In addition, the amount of the protein in the culture supernatant can be measured by an immunological method using an antibody against the protein (mentioned above). The present evaluation method enables analysis of NKT cells capable of maintaining the actions such as antitumor action.

While the functionality of NKT cells can be evaluated in various manners as mentioned above, the evaluation is preferably performed based on the measurement of the frequency of cells expressing the protein specific to functional NKT cells, since the number of functional NKT cells is determined more precisely.

The method of the present invention is useful for, for example, monitoring of an immune therapy, or determination of pathology (e.g., degree of progression of disease), prognosis and the like of an immunity-associated disease immune therapy. Since the method of the present invention enables functional analysis of NKT cells superior in the accuracy and reproducibility even in a human showing a decreased frequency of NKT cells in peripheral blood, the method is especially useful for monitoring an immune therapy and the like in a human showing a decreased frequency of NKT cells in peripheral blood . While the method of the present invention enables functional analysis of any low frequency NKT cells by appropriately setting the cultivation period of the above-mentioned (a), the functionality of low frequency NKT cells of, for example, not more than about 0.1%, preferably not more than about 0.05%, more preferably not more than about 0.01%, and yet more preferably not more than about 0.005%, can be analyzed appropriately (in the present specification, the NKT cell percentages (%) show the proportion of the number of NKT cells in cells in a lymphocyte fraction (T cell, B cell, NK cell, NKT cell)). Also, while the lower limit value of the frequency of NKT cells, whose functionality can be analyzed by the method of the present invention, is not particularly limited, the functionality of NKT cells can be analyzed when the value is not less than about 0.001%. As the immunity-associated diseases where the frequency of NKT cells in peripheral blood can be decreased, or where monitoring of immune therapy, or determination of pathology or prognosis is desired, cancer (e.g., prostate cancer, lung cancer, liver cancer, ovarian cancer, leukemia (e.g., chronic myeloid leukemia, acute myeloid leukemia, chronic lymphocyte leukemia, acute lymphocyte leukemia), MDS(myelodysplastic syndrome), malignant lymphoma, multiple myeloma), autoimmune diseases (e.g., systemic lupus erythematosus(SLE), chronic rheumatoid arthritis), allergic disease (e.g., rhinitis, bronchial asthma), virus infection (e.g., infection by a virus such as human immunodeficiency virus (HIV), hepatitis C virus and the like) and the like can be mentioned.

The present disclosure also provides a reagent for the analysis of human NKT cells. The reagent of the present disclosure comprises a mature dendritic cell derived from a heterologous animal. The mature dendritic cell derived from a heterologous animal is as mentioned above, and it may be the one that is stimulation-treated by a CDld ligand or the one that is untreated.

The present disclosure further provides a kit that can be used for the diagnosis or medical treatment, which kit enables convenient performance of the method of the present invention. The kit of the present disclosure can be, for example, the one comprising (a) a mature dendritic cell derived from a nonhuman animal, and (b) at least one reagent selected from the group consisting of reagents for selection of human mononuclear cells, reagents for measurement of the number of human NKT cells and reagents for measurement of a substance specific to human functional NKT cells. In addition, the kit of the present disclosure may further comprise a CD1d ligand. The kit of the present disclosure can further comprise an antibody against Vα24 and/or Vβ11, anti-CD3 antibody, anti-invariant NKT antibody and the like.

The reagent for fractionation treatment of human mononuclear cells is one that comprises an antibody against a marker molecule of human mature dendritic cells (mentioned above).

The reagent for measurement of the number of human NKT cells is one that comprises Vα24 antibody, Vβ11 antibody or anti-invariant NKT antibody, or a nucleic acid probe capable of detecting a transcription product encoding Vα24 or Vβ11 or plural amplifiable primers (e.g., primer set). For example, the reagent for measurement may be the one that comprises a primer for real-time RT-PCR, since it is beneficial to perform real-time RT-PCR.

The reagent for measurement of a substance specific to human functional NKT cells is one that comprises an antibody against the protein specific to human functional NKT cells (e.g., IFN-γ, perforin or granzyme B) (mentioned above), or a nucleic acid probe capable of detecting a transcription product encoding the substance or plural amplifiable primers (e.g., primer set). For example, the reagent for measurement may be the one that comprises a primer for real-time RT-PCR, since it is beneficial to perform real-time RT-PCR.

While the present invention is explained more specifically by the following Examples, the Examples are mere exemplification of the present invention, and do not limit the scope of the present invention in any way.

### Examples

### Material and method

### (1) Samples:

Peripheral blood mononuclear cells (PBMCs) were obtained from buffy coats that were provided by healthy blood donors (Tokyo Red Cross), and separated by Ficoll-Hypaque (Amersham Pharmacia Biotech, Uppsala, Sweden) density centrifugation. PBMCs were washed three times with PBS, and preserved in liquid nitrogen until the usage. Lymphocytes derived from patients were obtained from National Hospital Organization Kumamoto Medical Center (Kumamoto, Japan) and Kyoto University (Kyoto, Japan).

### (2) Reagents and antibodies:

Human rGM-CSF, IL-4, IL-1b and TNF-α were purchased from R&D systems (Minneapolis, MN). IL-2 was provided by Shionogi & Co., Ltd. (Osaka, Japan). PGE₂ was obtained from Sigma (Saint Louis, MO). α-Galactosylceramide (α-GalCer; 2S,3S,4R-1-O(α-galactopyranosyl)-2(N-hexacosanoylamino)-1,3,4-octadecanetriol) was diluted with 0.05% polysorbate-containing PBS. The following monoclonal antibodies (mABs) were obtained from BD PharMingen (San Diego, CA) (FITC-conjugated α-CD3, invariant NKT (6B11), α-CD4, and PE-conjugated α-CD8, α-CD40, α-CD80, α-CD83, α-CD86, invariant NKT (6B11), perforin, and APC-conjugated α-CD3, α-CD11c, α-CD154, α-IFN-γ, α-IL-4). α-Vα24-FITC and α-Vβ11-PE mAbs were purchased from Beckman Coulter (Fullerton, CA). For flow cytometry analysis, cells were incubated with mAb conjugates for 30 minutes, washed, and then analyzed by a FACS Calibur flow cytometer (Becton Dickinson, San Jose, CA).

### Example 1: Comparison of expression profiles of human/mouse dendritic cell (DC)

For a study of difference in NKT cell activation ability between human and mouse DC, expression profiles of human and mouse DC were studied first. Specifically, mature mouse DCs were obtained by cultivating mouse bone marrow cells in the presence of GM-CSF for 7 days, and then cultivating them while adding LPS for 1 day, and expressions of CD80, CD86 and CD40 were measured by flow cytometry. Mature human DCs were obtained by isolating monocytes, cultivating them in the presence of GM-CSF and IL-4, adding IL-1β, TNF-α and PGE2 6 days later, and cultivating them for 1 day, and expressions of CD80, CD86 and CD83 were measured by flow cytometry.

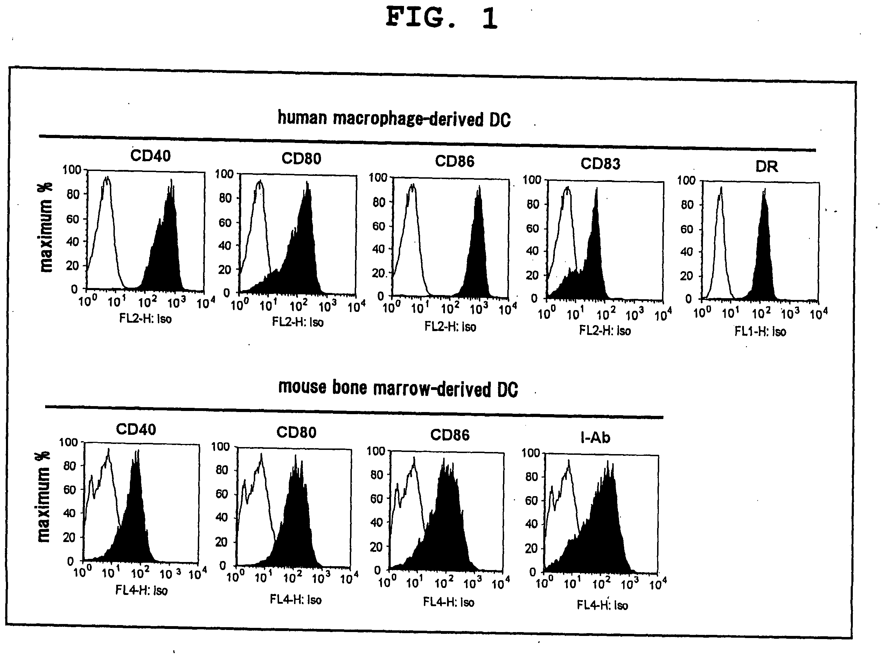

As a result, CD40, CD80, CD83, CD86 and DR were positive in human DCs, and CD40, CD80, CD86 and I-Ab were also positive in mouse DCs (Fig. 1).

From the foregoing, it was confirmed that both human and mouse DCs show similar expression profiles, and typical mature bone marrow dendritic cells can be obtained by the above-mentioned methodology.

### Example 2: Detection of high - low frequency primary NKT cell by ELISPOT assay in autologous, allogeneic or heterologous system using α-GalCer-loaded mature DC

In order to study whether activation of NKT cells in peripheral blood varies by DCs of different derivation, NKT cell activation in an autologous, allogeneic or heterologous system was studied by an ELISPOT assay with IFN-γ productivity as an index.

DCs were prepared as the following.

Human DCs were obtained by purifying CD14⁺ monocytes with magnetic beads (Miltenyi Biotec Inc. Auburn, CA) and cultivating them for 7 days in the presence of 500 U/ml recombinant human IL-4 and 1,000 U/ml recombinant human GM-CSF. α-GalCer (100 ng/ml) or the same amount of vehicle (DMSO, Sigma) was loaded onto DCs at the 5th day, given maturation stimuli (10 ng/ml IL-1β, 10 ng/ml TNFα, and 1 µg/ml PGE₂) at the 6th day, and recovered at the 7th day.

Bone marrow precursor cells were grown into DCs induced from mouse bone marrow in 5% FCS-containing RPMI-1640 using mouse GM-CSF. At the 6th day, α-GalCer (100 ng/ml) was added to immature bone marrow DCs, and cultivation was performed for 40 hr. For maturation of DCs, 100 ng/ml LPS was added at the 7th day, and cultivation was performed for 16 hr. Matured α-GalCer-pulsed DCs were recovered at the 8th day.

An ELISPOT assay was performed as the following.

For detection of IFN-γ-secreting cells, 96-well filtration plates (Millipore, Bedford, MA) were coated with mouse anti-human IFN-γ (10 µg/ml, Mabtech, Sweden). PBMCs (1×10⁶) were incubated in RPMI containing 5% human AB serum or 10% calf serum (complete medium or CM; GIBCO) in either presence or absence of α-GalCer (100 ng/ml). PMA (10 µg/ml) and ionomycin were used as positive controls. After the incubation, the plates were washed, and incubated with biotinated anti-human IFN-γ (1 µg/ml, Mabtech). Spot-forming cells (SFC) were quantified with a microscope. When the reactivity was not less than 10 SFC/well and not less than twice the background (without αGalCer), the assay was scored as positive. In order to compare naive NKT cell activation ability of different APCs (autologous, allogeneic and heterologous DC) in cultures, Mφ-depleted cells (1×10⁶/well) were cultivated with autologous α-GalCer or vehicle-loaded APCs (1×10⁵/well) in CM for 16 hr. α-GalCer-dependent IFN-γ-producing cells were measured by an ELISPOT assay.

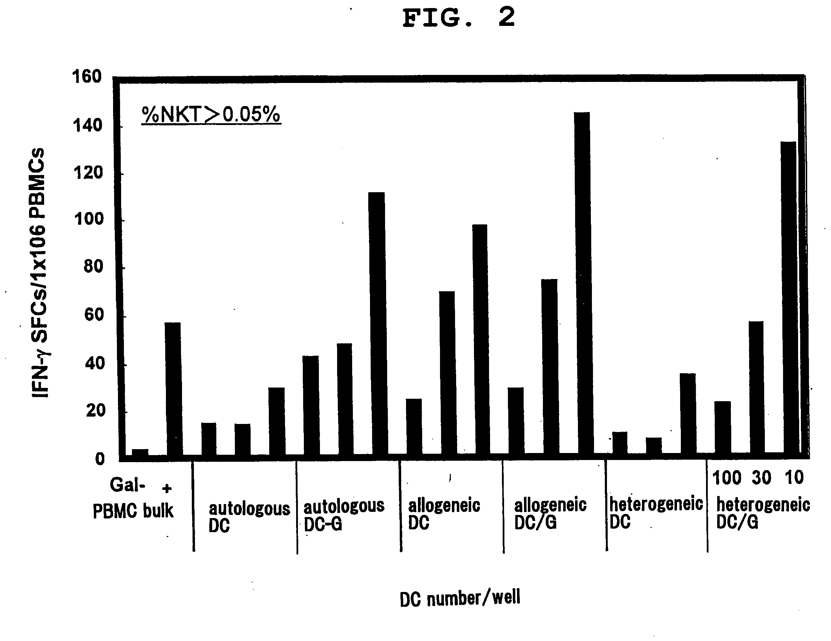

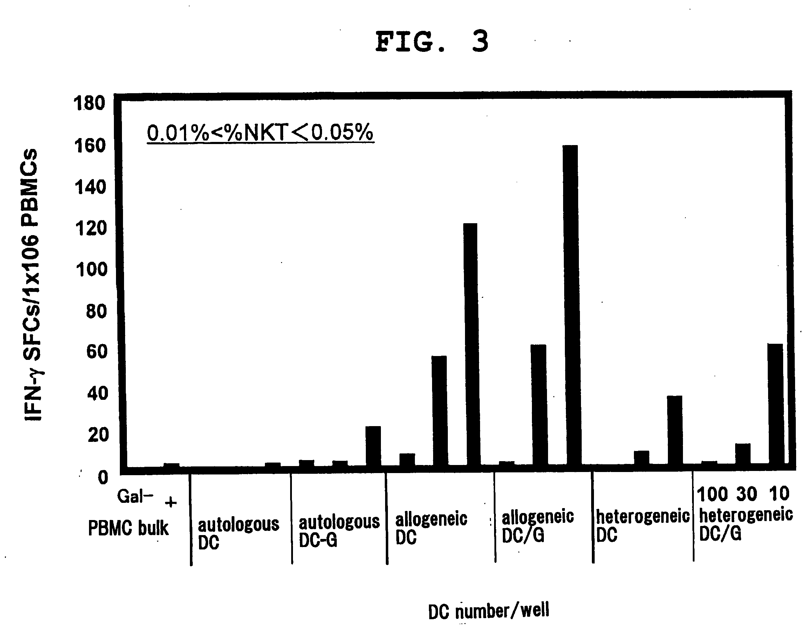

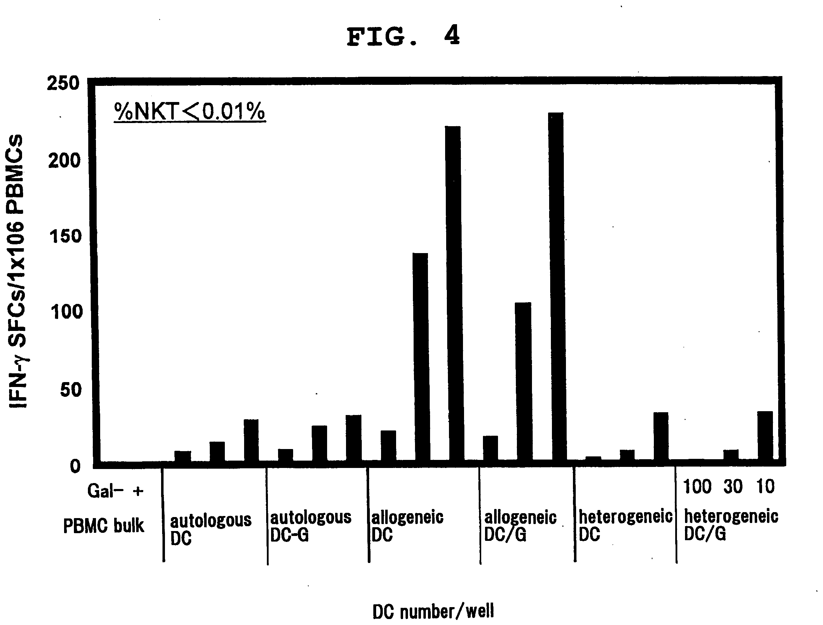

As a result, in the high frequency NKT cell-containing group (%NKT > 0.05%), activation of NKT cell was confirmed in autologous and heterologous DCs in an α-GalCer dependent manner (Fig. 2). Similarly, in the middle frequency NKT cell-containing group (0.01% < %NKT < 0.05%), activation of NKT cell was confirmed in an α-GalCer dependent manner, but the production amount was found to be decreased (Fig. 3). In the low frequency NKT cell-containing group (%NKT < 0.01%), α-GalCer-dependent NKT cell activation was not confirmed (Fig. 4) .

From the foregoing, it was found that an ELISPOT assay was effective in the high frequency - middle frequency NKT cell-containing groups, but not necessarily effective in the low frequency NKT cell-containing group.

### Example 3: Evaluation of human T cell response to autologous, allogeneic and heterologous DCs by mixed lymphocyte reaction (MLR)

In a system using allogeneic or heterologous DCs and human-derived PBMCs (allogeneic or heterologous system), the possibility was assumed that on evaluation of functionality of NKT cells contained in human-derived PBMCs, a side reaction (e.g., cytokine production) that was accompanied with non-NKT cell (particularly human T cell) response resulting from DCs had an impact on activation of human NKT cells. Therefore, it was studied whether or not T cell response that could induce such a side reaction in these systems would arise.

T cell response was evaluated by mixed lymphocyte reaction. Specifically, each type of mature DC (autologous, allogeneic and heterologous DCs) prepared by the same method as described in Example 2 was treated with radiation, put on a 96-well round bottom plate, added to 1×10⁵ Mφ-depleted PBMCs, and cultivated for 6 days. ³H-thymidine (1 µCi/well) was added in the final 16 hr.

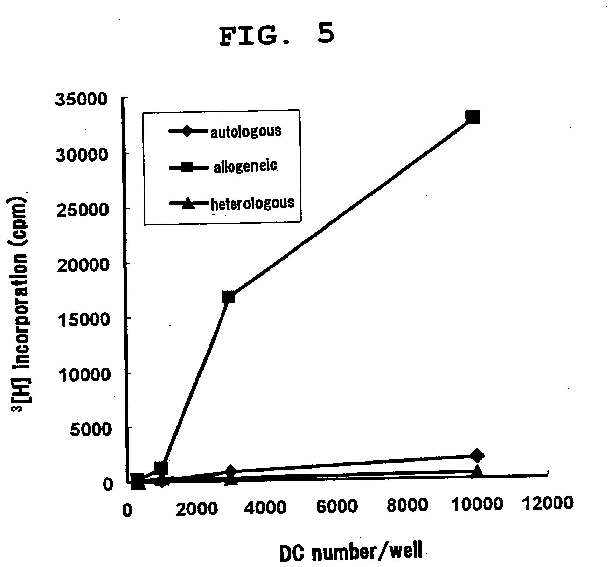

As a result, while allogeneic DCs strongly stimulated human T cells, autologous or heterologous DCs hardly stimulated them (Fig. 5). This suggests that T cell response that may affect activation of human NKT cells hardly arose in the heterologous system as well as in the autologous system.

From the foregoing, it was suggested that functionality of human NKT cell could be evaluated more genuinely by the autologous or heterologous system than by the allogeneic system.

### Example 4: Secondary activation of autologous or heterologous DC by activated NKT cell

The possibility was assumed that a DC was secondarily maturated or activated by an activated human NKT cell in the autologous or heterologous DC system, thereby evaluation of functionality of human NKT cell in these systems being influenced. Therefore, secondary alteration of DC by an activated human NKT cell was studied.

Immature human and mouse DCs were cocultivated with an activated human NKT cell line for 24 hr, after which alteration of phenotype (index of maturation) and cytokine production ability were measured. 5×10⁴ DCs derived from human or mouse were put on a 96-well plate, and cultivated with 5×10³ NKT cell line in the presence or absence of 100 ng/ml α-GalCer using a cytokine cocktail (PGE₂, IL-1b, TNF-α) or LPS (100 ng/ml; Sigma) for 24 hr. The supernatant was assayed using an ELISA kit for measurement of various cytokines (human IFN-γ, human IL-12p70 and human IL-10, and mouse IL-12p70 and mouse IL-10) (Opti EIA; BD PharMingen).

The NKT cell line used for the cocultivation was prepared as the following.

Mature DCs were stimulated for maturation, as well as pulsed using α-GalCer (100 ng/ml) for 48 hr in the 5th - 7th days of the cultivation. 1×10⁴ DCs pulsed with α-GalCer were added to the each well of 1×10⁵ Mφ-depleted PBMCs in the presence of recombinant IL-2 (100 U/ml). 10 - 14 Days later, Vα24⁺ NKT cells were stained with FITC-labeled anti-Vα24 mAb, and selected using anti-FITC magnetic beads. Vα24⁺ NKT cells were maintained as a cell line in CM in the presence of 100 U/ml IL-2, and restimulated by PBMCs that had been irradiated in the presence of α-GalCer.

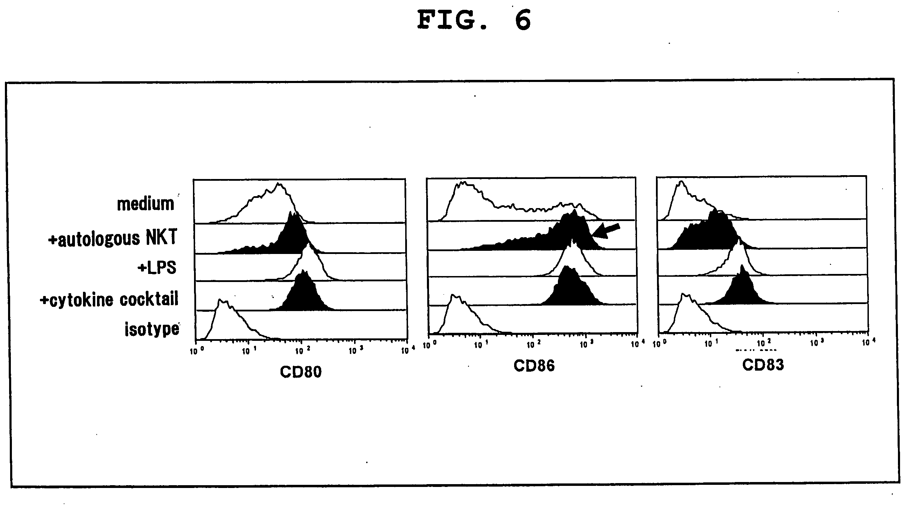

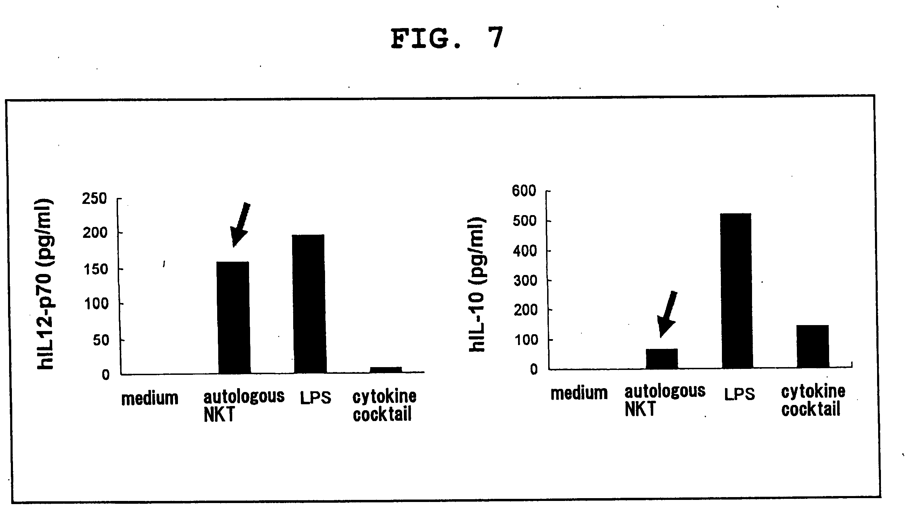

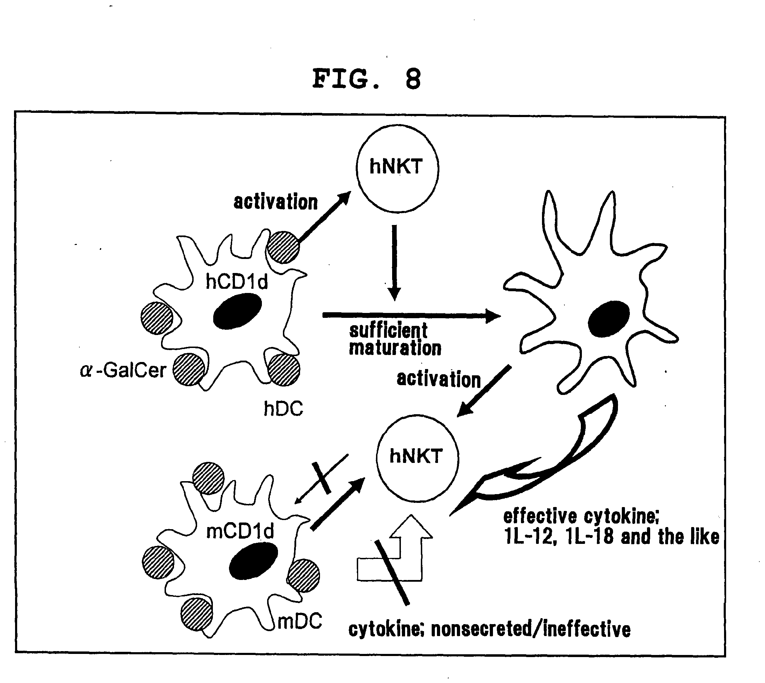

As a result, autologous immature DCs were maturated (Fig. 6), but heterologous immature DCs were not (data not shown). Also, as to cytokine production, IL-12 production was found only in the autologous DCs (Fig. 7), but not in the heterologous DCs (data not shown). This suggests that consideration of NKT cell activation by a secondary cytokine produced by DCs is not necessary when heterologous DCs are used (Fig. 8).

From the foregoing, it was suggested that functionality of human NKT cell can be evaluated more genuinely by the heterologous system than by the autologous system.

### Example 5: Activation of human NKT cell by α-GalCer-pulsed heterologous DC

In order to clarify that a human NKT cell can be specifically activated by a heterologous DC, human NKT cells were isolated, cocultivated with α-GalCer-pulsed autologous or heterologous DCs, after which the culture supernatants were collected, and cytokine production was measured by the ELISA method.

After growth of the NKT cell with α-GalCer and IL-2, the cells were sorted using FACS Vantage with anti-invariant NKT antibodies, and cocultivated with α-GalCer-pulsed heterologous or autologous DCs, after which IFN-γ was quantified by the ELISA method.

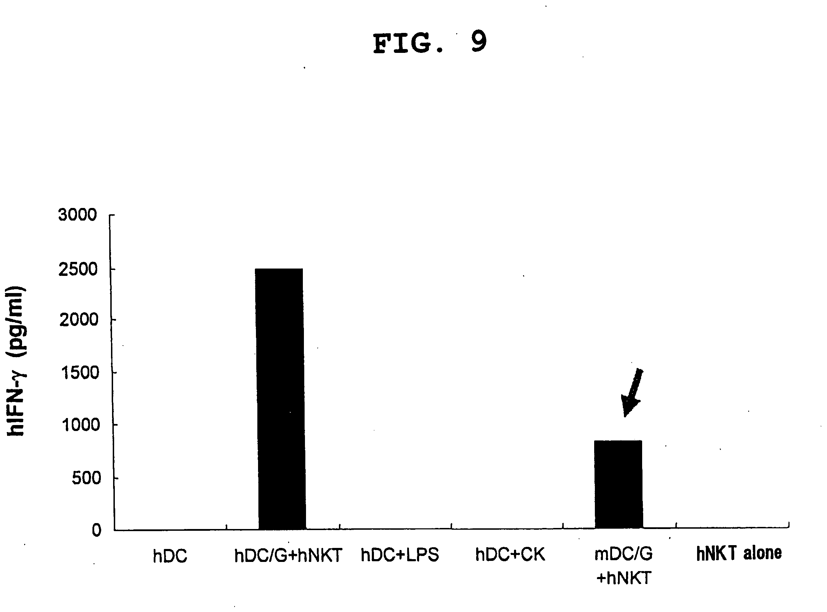

As a result, IFN-γ production was confirmed in the heterologous system, though it was lower than in the autologous system (Fig. 9).

From the foregoing, it was clarified that α-GalCer-dependent immune response could be utilized for evaluation of functionality of human NKT cells in the heterologous system.

### Example 6: Antigen-specific growth of NKT cell by heterologous DC

After serial dilution of the NKT cell line that was prepared by the method described in Example 4, the diluted solutions were mixed with NKT cell-removed PBMCs, cocultivated with heterologous DCs in the presence of an antigen (α-GalCel), and the growth rates were calculated 10 days later.

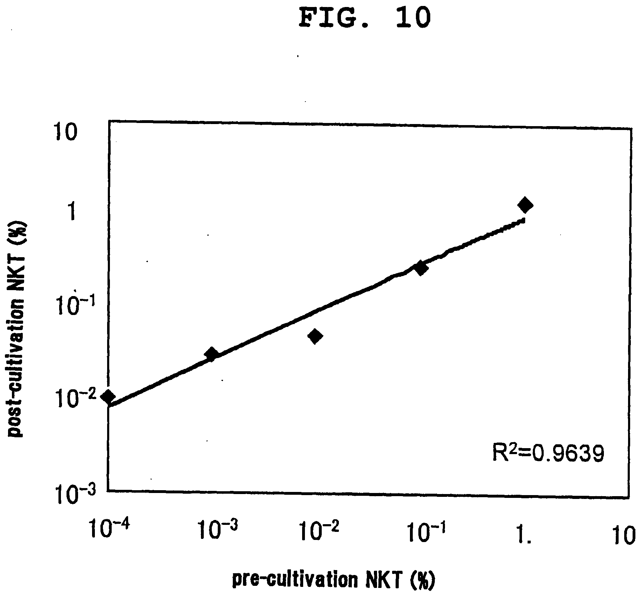

As a result, human NKT cells were antigen-specifically grown by heterologous DCs irrespective of the number of NKT cells prior to the start of the cultivation while maintaining the relationship of direct proportion between before and after the cultivation (Fig. 10). Furthermore, after the growth culture, NKT cells with 0.01 - 0.001% frequency were detectable.

From the foregoing, it was suggested that in the heterologous system NKT cells contained in peripheral blood were grown by setting an appropriate cultivation period, thereby enabling detection of low frequency NKT cell, which was impossible by conventional ELISPOT assays.

### Example 7: Analysis of NKT cell contained in peripheral blood derived from a healthy subject

Based on the results from the above-mentioned Example, in order to study whether or not the function of NKT cell contained in peripheral blood of a healthy subject, which comprised a relatively great number of NKT cells, could be skillfully analyzed by the protocol for functional analysis of NKT cell with heterologous system developed by the present inventors, the number of NKT cells and the frequency of NKT cells having an cytokine production ability were measured before and after the cultivation (growth) by the protocol of the present inventors.

The protocol of the present inventors was the following (1) - (3).

### (1) Preparation of mature mouse DC

At first, mouse DCs were induced from mouse bone marrow cells. Mouse bone marrow cells were cultivated in 5% FCS-containing RPMI-1640 in the presence of mouse GM-CSF under 5% CO₂ at 37°C for 5 days.

Subsequently, immature mouse DCs were maturated. To the culture obtained as mentioned above (containing immature mouse DCs), α-GalCer (100 ng/ml) was added at the 6th day, and the culture was cultivated for 40 hr. For an acieration of the maturation, 100 ng/ml LPS was added at the 7th day, and the culture was cultivated for 16 hr. Thus obtained α-GalCer-pulsed mature mouse DCs were recovered at the 8th day.

### (2) Preparation of Mφ-depleted PBMC derived from a tested sample

PBMCs were obtained by blood drawing from healthy subjects, and Mφ-depleted PBMCs were obtained by removing macrophages (Mφ) using an anti-CD14 antibody (simultaneously using a CD11c antibody as necessary) and magnetic beads.

### (3) Cocultivation of Mφ-depleted PBMC and mature mouse DC

α-GalCer-pulsed mature mouse DCs (5x10⁴ cells/ml) obtained by the above-mentioned (1) and Mφ-depleted PBMCs (5x10⁵ cells/ml) obtained by the above-mentioned (2) were cultivated in 10% FCS-containing RPMI-1640 in the presence of recombinant human IL-2 (100 U/ml) under 5% CO₂ at 37°C for 9 days.

After cultivation in accordance with the protocol of the above-mentioned (1) - (3), the number of NKT cells and the frequency of NKT cells having an cytokine production ability were measured by flow cytometry and intracellular staining. As comparative controls, similar experiments were performed using a group cultivated with IL-2 alone and an α-GalCer-nonpulsed heterologous DC group.

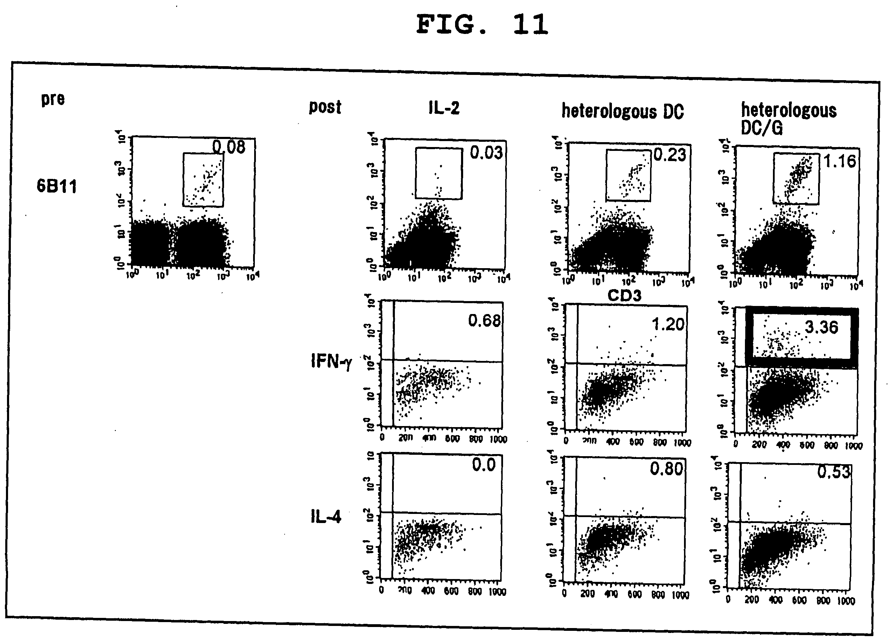

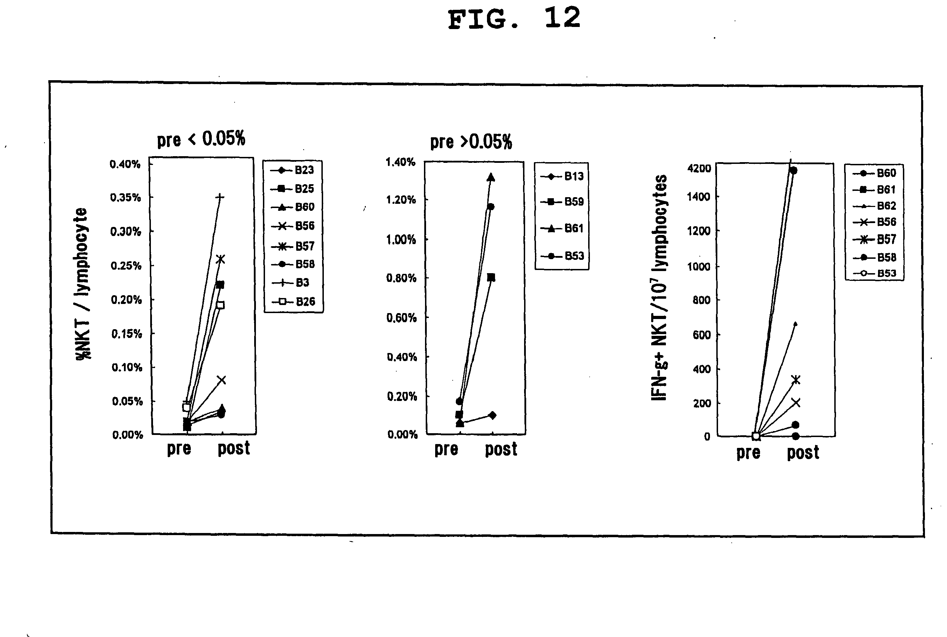

As a result, the α-GalCer-dependent growth of human NKT cells and the cytokine production caused by heterologous DCs were confirmed (Figs. 11 - 12).

From the foregoing, it was suggested that activation of NKT cells in peripheral blood from a healthy subject (or a tested sample containing a relatively great number of NKT cells) can be analyzed by the protocol developed by the present inventors.

### Example 8: Analysis of NKT cell contained in peripheral blood derived from a chronic myeloid leukemia (CML) patient

Then, the number of NKT cells and the frequency of NKT cells having a cytokine production ability were measured before and after the cultivation (growth) by the protocol of the present inventors using peripheral blood derived from CML patients, where the number of NKT cells was small and a decrease in the function of antigen presenting cells was suggested. For CML patients, patients receiving a treatment with imatinib were adopted as targets.

After cultivation in accordance with the protocol of the above-mentioned (1) - (3) using peripheral blood derived from CML patients who were receiving a treatment with imatinib, NKT cell frequency and cytokine production ability were measured by flow cytometry and intracellular staining. As comparative controls, similar experiments were performed using a group cultivated with IL-2 alone and an α-GalCer-nonpulsed heterologous DC group.

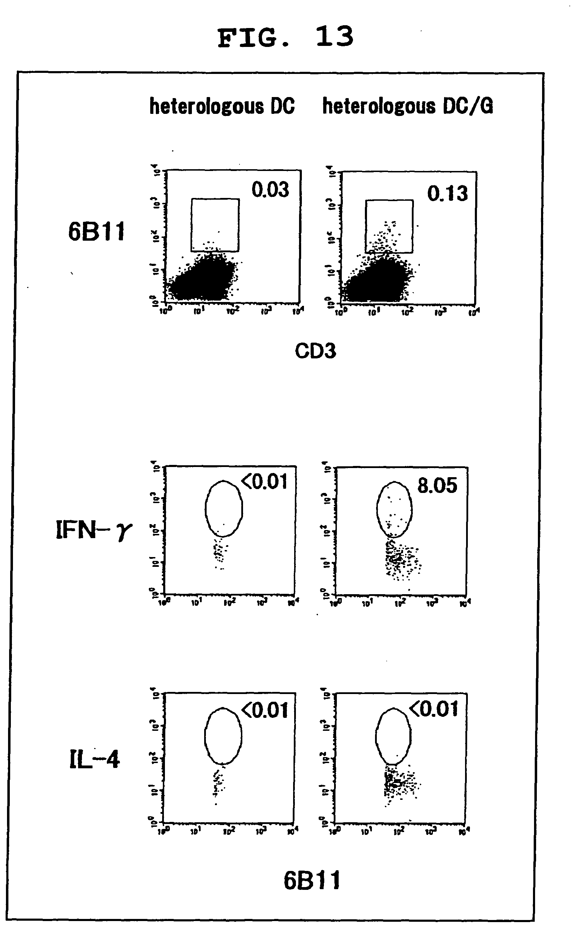

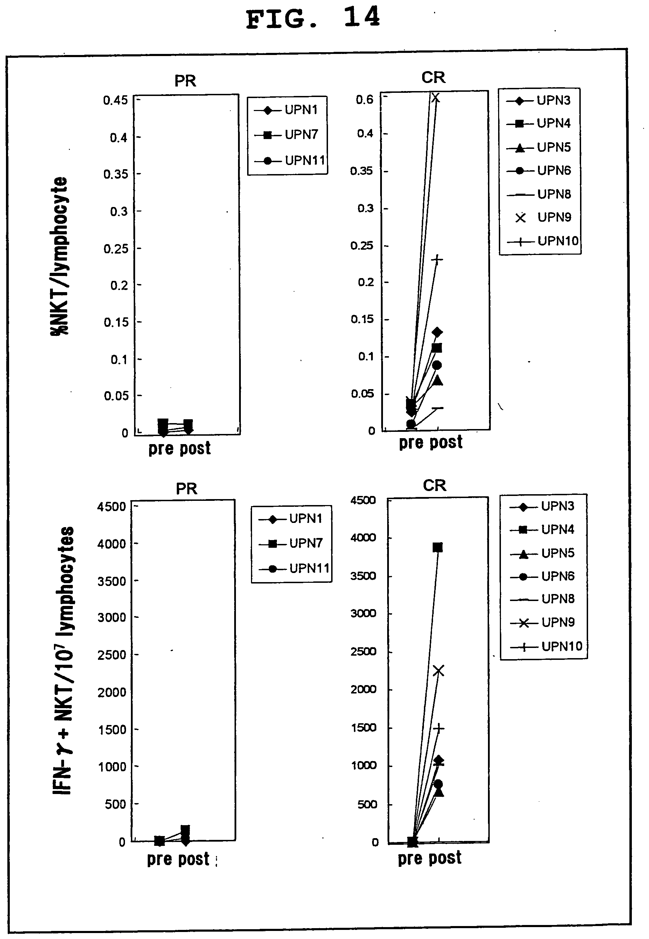

As a result, the heterologous system was able to detect peripheral blood NKT cells from such patients (%NKT ≦ 0.02%), which were not identifiable by the ELISPOT method (Figs. 13 - 14). Also, functions such as growth ability, cytokine production ability and the like were preserved in CR patients, though the number of NKT cells was decreased. On the other hand, the number of NKT cells was decreased also in PR patients, but functions such as growth ability, cytokine production ability and the like were not necessarily preserved.

From the foregoing, it has been suggested that the protocol of the present inventors can skillfully analyze the activation of NKT cell in the peripheral blood containing low frequency NKT cells, and is useful for monitoring an immune therapy and the like.

### Industrial Applicability

The present invention enables functional analysis of low frequency NKT cell, is independent of the function of autologous APC, can avoid an influence of a secondary factor and the like, and enables a functional analysis of NKT cell, which is superior to conventional methods.

## Claims

1. A method of functional analysis of NKT cell in human, which comprises:
(a) cocultivating a mononuclear cell population containing a human NKT cell derived from human peripheral blood with a mature dendritic cell derived from a rodent, wherein the mature dendritic cell is a cell pulsed with a CD1d ligand, and
(b) evaluating the functionality of NKT cell based on the number of NKT cells and/or a protein specific to functional NKT cells or a transcription product encoding same as an index.

2. The method of claim 1, wherein the CD1d ligand is α-GalCer.

3. The method of claim 1, wherein the mononuclear cell population is a macrophage-depleted mononuclear cell population.

4. The method of claim 1, wherein the substance specific to functional NKT cells is IFN-γ;

5. The method of claim 1, wherein the evaluation of the functionality of NKT cell based on the number of NKT cells and/or a protein specific to functional NKT cells or a transcription product encoding same as an index is based on a measurement of frequency of cell that expresses the protein specific to functional NKT cells or the transcription product encoding same.

6. The method of claim 1, wherein the rodent is a mouse.

7. The method of claim 1, which is an analysis method of humans that show NKT cell frequency of about 0.05% - about 0.001% in peripheral blood.

8. The method of claim 1, which is a method for monitoring an immune therapy, or determination of the pathology or prognosis in a patient with a disease selected from the group consisting of cancer, autoimmune disease, allergic disease and virus infection.

## Patentansprüche

1. Verfahren zur funktionellen Analyse von NKT-Zelle beim Menschen, das Folgendes umfasst:
(a) gemeinsames Kultivieren einer mononukleären Zellpopulation, die menschliche NKT-Zelle beinhaltet abgeleitet von menschlichem peripherem Blut mit einer reifen dendritischen Zelle abgeleitet von einem Nagetier, wobei die reife dendritische Zelle eine Zelle ist, die mit einem CD1d-Liganden gepulst ist, und
(b) Bewerten der Funktionalität von NKT-Zelle beruhend auf der Anzahl von NKT-Zellen und/oder eines Proteins, das spezifisch für funktionelle NKT-Zellen ist, oder ein Transkriptionsprodukt, das es als einen Index kodiert.

2. Verfahren nach Anspruch 1, wobei der CD1d-Ligand α-GalCerist.

3. Verfahren nach Anspruch 1, wobei die mononukleäre Zellpopulation eine Makrophagen-depletierte mononukleäre Zellpopulation ist.

4. Verfahren nach Anspruch 1, wobei die spezifische Substanz für funktionelle NKT-Zellen IFN-γ ist.

5. Verfahren nach Anspruch 1, die Bewertung der Funktionalität von NKT-Zelle beruhend auf der Anzahl von NKT-Zellen und/oder eines Proteins, das spezifisch für funktionelle NKT-Zellen ist, oder ein Transkriptionsprodukt, das gleich wie ein Index kodiert, auf einer Messung der Häufigkeit von Zellen beruht, die das Protein exprimieren, das spezifisch für funktionelle NKT-Zellen ist, oder das Transkriptionsprodukt, das dieses kodiert.

6. Verfahren nach Anspruch 1, wobei das Nagetier eine Maus ist.

7. Verfahren nach Anspruch 1, das ein Analyseverfahren von Menschen ist, die eine NKT-Zellhäufigkeit von ungefähr 0,05 % - ungefähr 0,001 % in peripherem Blut zeigt.

8. Verfahren nach Anspruch 1, das ein Verfahren zum Überwachen einer Immuntherapie, oder zur Bestimmung der Pathologie oder Prognose bei einem Patienten mit einer Krankheit ist, die ausgewählt ist aus der Gruppe bestehend aus Krebs, Autoimmunkrankheit, allergischer Krankheit und Virusinfektion.

## Revendications

1. Procédé d'analyse fonctionnelle d'une cellule NKT chez l'humain, qui comprend :
(a) la coculture d'une population de cellules mononucléés contenant une cellule NKT humaine dérivée de sang périphérique humain avec une cellule dendritique mature dérivée d'un rongeur, la cellule dendritique mature étant une cellule pulsée avec un ligand CD1d, et
(b) l'évaluation de la fonctionnalité de la cellule NKT en fonction du nombre de cellules NKT et/ou d'une protéine spécifique aux cellules NKT fonctionnelles ou d'un produit de transcription codant la même chose qu'un indice.

2. Procédé selon la revendication 1, dans lequel le ligand CD1d est le α-GalCer.

3. Procédé selon la revendication 1, dans lequel la population de cellules mononucléées est une population de cellules mononucléées appauvrie en macrophages.

4. Procédé selon la revendication 1, dans lequel la substance spécifique aux cellules NKT fonctionnelles est l'IFN-γ.

5. Procédé selon la revendication 1, dans lequel l'évaluation de la fonctionnalité d'une cellule NKT en fonction du nombre de cellules NKT et/ou d'une protéine spécifique à des cellules NKT fonctionnelles ou d'un produit de transcription codant la même chose qu'un indice est basée sur une mesure de la fréquence d'une cellule qui exprime la protéine spécifique aux cellules NKT fonctionnelles ou le produit de transcription codant celui-ci.

6. Procédé selon la revendication 1, dans lequel le rongeur est une souris.

7. Procédé selon la revendication 1, qui est un procédé d'analyse d'humains qui présentent une fréquence de cellules NKT d'environ 0,05 % à environ 0,001 % dans le sang périphérique.

8. Procédé selon la revendication 1, qui est un procédé de surveillance d'une thérapie immunitaire, ou de détermination de la pathologie ou du pronostique chez un patient présentant un trouble choisi parmi le groupe constitué du cancer, d'une maladie auto-immune, d'une maladie allergique et d'une infection virale.
